(19) Europäisches Patentamt
European Patent Office
Office européen des brevets

(11) Veröffentlichungsnummer: **0 079 537**
**A1**

(12) **EUROPÄISCHE PATENTANMELDUNG**

(21) Anmeldenummer: 82110188.8

(22) Anmeldetag: 05.11.82

(51) Int. Cl.³: C 07 C 131/00
C 07 D 295/20, C 07 D 265/30
A 01 N 37/18

(30) Priorität: 10.11.81 DE 3144600

(43) Veröffentlichungstag der Anmeldung: 25.05.83 Patentblatt 83/21

(84) Benannte Vertragsstaaten: BE CH DE FR GB IT LI NL

(71) Anmelder: HOECHST AKTIENGESELLSCHAFT
Postfach 80 03 20
D-6230 Frankfurt/Main 80(DE)

(72) Erfinder: Boesenberg, Heinz, Dr.
Am Dachsgraben 4
D-6238 Hofheim am Taunus(DE)

(72) Erfinder: Bauer, Klaus, Dr.
Kolpingstrasse 7
D-6054 Rodgau(DE)

(72) Erfinder: Bieringer, Hermann, Dr.
Eichenweg 26
D-6239 Eppstein/Taunus(DE)

(54) **Carbamoyloxime, Verfahren zu ihrer Herstellung und ihre Verwendung als Herbizide.**

(57) Verbindungen der Formel I

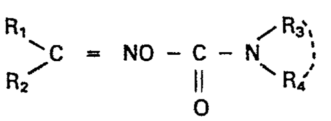

(I)

worin $R_1$ (substituiertes) ($C_1$-$C_6$)Alkyl, (substituiertes) ($C_3$-$C_8$)Cycloalkyl oder (substituiertes)Phenyl, $R_2$ ($C_3$-$C_6$)Alkyl oder Alkox-($C_3$-$C_6$)alkyl, $R_3$ und $R_4$ u.a. Alkyl oder Phenyl oder zusammen mit dem Stickstoff einen Heterocyclus bedeuten, sind wirksame Herbizide.

EP 0 079 537 A1

## Carbamoyloxime, Verfahren zu ihrer Herstellung und ihre Verwendung als Herbizide

Gegenstand der vorliegenden Erfindung sind neue Carbamoyloxime der allgemeinen Formel I, Verfahren zu ihrer Herstellung und ihre Verwendung als Herbizide.

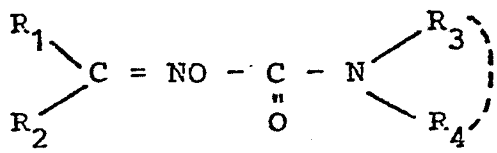

(I)

In Formel I bedeuten:

$R_1$ $(C_1-C_6)$-Alkyl, das geradkettig oder verzweigt ist und durch $(C_1-C_4)$-Alkoxy oder $(C_1-C_4)$-Alkoxycarbonyl substituiert sein kann, $(C_3-C_6)$-Cycloalkyl, das durch $(C_1-C_4)$-Alkyl oder $(C_1-C_4)$-Alkoxy substituiert sein kann, oder Phenyl, das durch $(C_1-C_4)$-Alkyl, $(C_1-C_4)$-Alkoxy, Cl oder Br substituiert sein kann,

$R_2$ $(C_3-C_6)$-Alkyl, das geradkettig oder verzweigt ist oder $(C_1-C_4)$-Alkoxy-$(C_3-C_6)$-alkyl,

$R_3$ $(C_1-C_6)$-Alkyl, das durch $(C_1-C_4)$-Alkoxy oder $(C_1-C_4)$-Alkoxycarbonyl substituiert sein kann; $(C_1-C_6)$-Alkenyl, $(C_1-C_6)$-Alkinyl, $(C_3-C_6)$-Cycloalkyl, das durch $(C_1-C_4)$-Alkyl oder $(C_1-C_4)$-Alkoxy substituiert sein kann,

$R_4$ $(C_1-C_{12})$-Alkyl, das durch $(C_1-C_4)$-Alkoxy oder $(C_1-C_4)$-Alkoxycarbonyl substituiert sein kann; $(C_1-C_6)$-Alkenyl; Phenyl-, Benzyl- oder Phenethyl, die im aromatischen Kern durch Halogen, $(C_1-C_4)$-Alkyl, $(C_1-C_4)$-Alkoxy, $(C_1-C_4)$-Alkoxycarbonyl, Cyan- oder Trifluormethyl substituiert sein können, oder

$R_3$ und $R_4$ zusammen mit dem benachbarten Stickstoffatom einen 3 - 6-gliedrigen heterocyclischen Ring, der 1 - 2-fach durch $(C_1-C_4)$-Alkyl oder $(C_1-C_4)$-Alkoxy substituiert sein kann und/oder in dem ein oder zwei Ringkohlenstoffatome durch -O-, -S-, C=O, -NH- oder N-$CH_3$ ersetzt sein können.

Unter den Verbindungen der Formel I sind solche bevorzugt, in denen der Rest $R_2$ verzweigt ist und insbesondere Isopropyl, sek. Butyl oder tert. Butyl bedeutet. Für $R_1$ seien außer den in den Beispielen erwähnten Resten noch die folgenden beispielsweise genannt: $-CH_2COOCH_3$, $-CH_2COOC_2H_5$, $-CH_2-OCH_3$, $-CH_2-OC_2H_5$, $CH_2-OC_4H_9(n)$, p-Tolyl, p-Chlorphenyl, p-Methoxyphenyl.

Die Verbindungen der Formel I lassen sich herstellen, indem man

a) Verbindungen der Formel

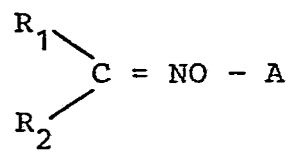

(II)

mit Verbindungen der Formel

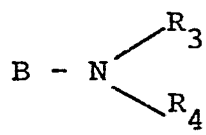

(III),

worin jeweils einer der Reste A und B die Gruppe Hal-CO- und der andere Wasserstoff oder ein Alkali- oder Ammoniumkation darstellt, gegebenenfalls in Anwesenheit von Säureakzeptoren umsetzt, oder

b) Verbindungen der Formel

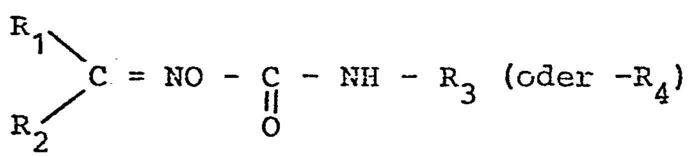

(IV)

alkyliert.

a) Gemäß der Varianten a) kann man sowohl Ketoxime (A=H) bzw. deren Salze mit Carbamoylhalogeniden (B=Hal-CO-) als auch Halogenformyloxime (A = -CO-Hal, bes. -CO-Cl) mit Aminen (B=H) oder deren Salzen umsetzen. Die Temperaturen liegen bei der ersten Alternative zwischen 0 und 120°C, vorzugsweise bei 20 - 80°C, bei der zweiten

Alternative (über die Chlorformyloxime) arbeitet man zweckmäßig bei -20 bis +50°C, vorzugsweise bei -10 bis +25°C. Die Darstellung der Oxime geschieht auf dem Fachmann geläufige Weise /siehe z.B. Houben-Weyl, "Methoden der organischen Chemie", Bd. X/4 (1968), S. 55 - 66/. Auch die Herstellung der benötigten Carbamoyl-halogenide ist in der Literatur mehrfach beschrieben /vgl. Houben-Weyl "Methoden der organischen Chemie", Bd. VIII (1952), S. 117 - 118; Bull. Soc. Chim. 31, 689 (1904); J. Chem. Soc. 1947, 313/. Halogenformyloxime können gemäß Zeitschrift für Chemie 7, 344-345 (1967) hergestellt werden. Amine sind literaturbekannte Verbindungen bzw. können auf literaturbekannte Weise erhalten werden.

Wenn man von den freien Ketoximen (A=H) oder Aminen (B=H) als Ausgangsstoffen ausgeht, ist es erforderlich, ein säurebindendes Mittel zur Neutralisation des freiwerdenden Halogenwasserstoffs einzusetzen. Als solche eignen sich basisch wirkende Agentien, wie NaOH, KOH, $NaHCO_3$, $Na_2CO_3$ oder $K_2CO_3$. Auch tertiäre Amine, wie Triethylamin oder Pyridin oder ein Überschuß des als Ausgangsstoff verwendeten Amins können als säurebindende Mittel eingesetzt werden.

Zweckmäßig führt man die Reaktion in einem gegenüber den Reaktanden inerten Lösungsmittel durch. Hierzu eignen sich Kohlenwasserstoffe, wie Hexan, Benzol, Toluol oder Xylol, chlorierte Kohlenwasserstoffe, wie Methylenchlorid, Chloroform, Tetrachlormethan oder Chlorbenzol; Ether, wie Diethylether, Glykol-dimethylether, Tetrahydrofuran oder Dioxan oder Ester wie Essigsäureethylester; auch polare Lösungsmittel wie Wasser, Acetonitril, Dimethylformamid, Dimethyl-sulfoxid oder Gemische der vorgenannten Lösungsmittel können eingesetzt werden.

b) Die gemäß Variante b) als Ausgangsstoffe in Betracht

kommenden Monoalkyl-carbamoyloxime können durch Umsetzung von Ketoximen (II; A=H) mit Isocyanaten der Formel $O=C=N-R_3$ (bzw. $-R_4$) hergestellt werden. Die Darstellung solcher Isocyanate kann auf den in Houben-Weyl VIII (1952), S. 119-128 angegebenen Wegen erfolgen. Ihre Alkylierung erfolgt in bekannter Weise mit üblichen Alkylierungsmitteln wie Methyljodid oder Dimethyl-(Diethyl-)sulfat. Die Reaktionsbedingungen entsprechen denjenigen des Verfahrens a).

Bestimmte Mono- und Dialkylcarbamate von substituierten Aldoximen und Ketoximen sind im Pflanzenschutz als Insektizide, Alkarizide und Reifebeschleuniger bekannt (z.B. US-PS 3 217 037, 3 576 834, JA-OS 72 41011, DE-OS 23 38010). Ferner werden Monoarylcarbamate von Ketoximen mit herbizider Wirkung in DB-PS 1 024 746 beschrieben. Bekannt sind ferner verschiedene Dialkylcarbamate des Acetonoxims (Zeitschr. f. Chemie 7, 344 (1967)).

Carbamoyl-Ketoxime, die sich von Ketonen mit mindestens einer längerkettigen ($C\geq3$) Alkylgruppe ableiten, sind bisher nicht bekannt geworden. Ebensowenig war bekannt, daß Carbamoyloxime mit einem sekundären Carbamatrest herbizide Eigenschaften besitzen. Es war daher überraschend, daß die erfindungsgemäßen Carbamoyl-Ketoxime eine ausgezeichnete herbizide Wirkung zeigen.

Sie eignen sich nicht nur als Totalherbizide, sondern auch zur selektiven Bekämpfung von zahlreichen annuellen und perennierenden Unkräutern und Ungräsern in landwirtschaftlich bedeutenden Kulturen wie etreide, Mais, Reis, Sojabohne, Baumwolle, Zuckerrüber und Raps. Besonders geeignet sind sie für die Anwendung im Vorauflaufverfahren. Eine Einarbeitung der ausgebrachten Verbindungen in den Boden kann dabei unter Umständen die Wirkung noch steigern.

Gegenstand der Erfindung sind daher auch herbizide Mittel, die gekennzeichnet sind durch einen wirksamen Gehalt an einer oder mehreren Verbindungen der Formel I.

Im allgemeinen enthalten die erfindungsgemäßen Mittel die Wirkstoffe der Formel I zu 2 - 80 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenyl-sulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden:

Alkylsulfonsaure Calziumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxethylen-sorbitan-Fettsäureester oder Polyoxethylensorbitester.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

Granulate können entweder durch Verdüsen des Wirkstoffes

0079537

auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen auf die Oberfläche von Trägerstoffen, wie Sand, Kaolinite, oder von granuliertem Inertmaterial. Auch können geeignete Wirkstoffe in der für die Herstellung von Düngemittelgranalien üblichen Weise gewünschtenfalls in Mischung mit Düngemitteln - hergestellt werden.

Bei herbiziden Mitteln können die Konzentrationen der Wirkstoffe in den handelsüblichen Formulierungen verschieden sein.

In Spritzpulvern variiert die Wirkstoffkonzentration z.B. zwischen etwa 10 % und 80 %, der Rest besteht aus den oben angegebenen Formulierungszusätzen. Bei emulgierbaren Konzentraten kann die Wirkstoffkonzentration gleichfalls etwa 10 % bis 80 % betragen.

Staubförmige Formulierungen enthalten meistens 5 - 20 % an Wirkstoff, versprühbare Lösungen etwa 2 - 20 %.
Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Zur Anwendung werden die handelsüblichen Konzentrate gegebenenfalls in üblicher Weise verdünnt, z.B. bei Spritzpulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt.
Mit den äußeren Bedingungen wie Temperatur, Feuchtigkeit, u.a. variiert die erforderliche Aufwandmenge.
Sie beträgt im allgemeinen etwa 0,5 - 5 kg/ha, vorzugsweise etwa 1 - 2,5 kg/ha Wirkstoff. Der erfindungsgemäße

Wirkstoff kann mit anderen Herbiziden und Bodeninsektiziden kombiniert werden.

## Herstellungsbeispiele

### Beispiel 1

N,N-Dimethylcarbamoyl-2,4-dimethylpentanon-(3)-oxim

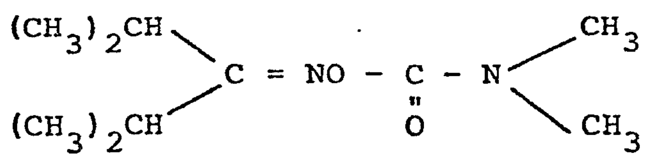

Zu einer Aufschlämmung von 10,0 g (0,33 mol) Natriumhydrid (80 %ige Öl-Suspension) in 150 ml absolutem Dioxan wurden innerhalb von 45 Minuten unter Rühren und Überlagerung mit einem schwachen Strom getrocknetem Stickstoff 38,8 g (0,3 mol) 2,4-Dimethyl-pentanon-(3)-oxim, gelöst in 50 ml absol. Dioxan, tropfenweise zugegeben, wobei die Temperatur von 21° auf 42°C anstieg. Zur Vervollständigung der Salzbildung wurde 30 Minuten bei 50°C nachgerührt. Dazu wurden bei gleichbleibender Temperatur 34,4 g (0,32 mol) N,N-Dimethyl-carbamoylchlorid, verdünnt mit 30 ml absol. Dioxan, tropfenweise zugefügt und 5 Stunden bei 80°C nachgerührt. Danach wurde das Gemisch auf Raumtemperatur abgekühlt, vom ausgefallenen Kochsalz abgesaugt, mit wenig frischem Dioxan nachgewaschen und das Lösungsmittel im Vakuum abdestilliert. Das zurückbleibende Öl wurde in 250 ml Methylenchlorid aufgenommen, die Lösung 2 mal mit je 50 ml Wasser gewaschen, über $Na_2SO_4$ getrocknet und das Lösungsmittel abermals abdestilliert. Es hinterblieb ein fast farbloses Öl, das im Hochvakuum von anhaftenden Lösungsmittelspuren befreit wurde. Ausbeute 50,4 g (83,9 % der Theorie), $n_D^{25}$ = 1,4613.

Beispiel 2

(N-Methyl-N-phenyl-carbamoyl)-methyl-tert.butyl-ketoxim

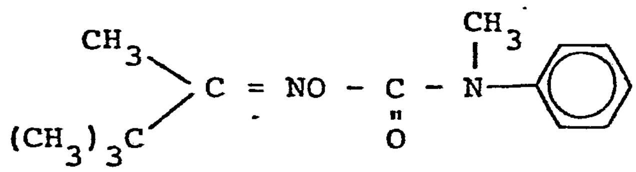

In eine auf -15°C gekühlte Lösung von 30,0 g (0,3 mol) Phosgen in 200 ml wasserfreiem Toluol, werden unter Rühren 17,3 g (0,15 mol) Methyl-tert.butyl-ketoxim, gelöst in 50 ml absol. Toluol, langsam eingetropft, so daß die Temperatur -5°C nicht übersteigt. Das Reaktionsgemisch wird 30 Minuten im Kältebad und 1 Stunde bei Raumtemperatur gerührt, danach die Hauptmenge des überschüssigen Phosgens und der gelöste Chlorwasserstoff durch einen trockenen Stickstoffatom aus der Lösung ausgeblasen, das Toluol bei 50°C im Wasserstrahlvakuum abdestilliert und durch 100 ml frisches Toluol ersetzt, das in gleicher Weise destilliert wurde.

In die mit Toluol auf 150 ml aufgefüllte und erneut auf -10°C gekühlte Lösung des rohen Chlor-formyloxims wird nun ein Gemisch aus 17,2 g (0,16 mol) N-Methyl-anilin und 17,2 g (0,17 mol) Triethylamin, verdünnt mit 50 ml Toluol tropfenweise zugegeben und das Reaktionsgemisch anschließend 30 Minuten unter Eiskühlung und 1 Stunde bei Raumtemperatur nachgerührt.

Nach dem Abfiltrieren des ausgefallenen Triethylamin-hydrochlorids und Einengen des Filtrats im Vakuum kristallisiert das Carbamat und wird aus Petrolether umkristallisiert. Ausbeute: 25,8 g (69,3 % der Theorie), Fp. 94 - 96°C.

In analoger Weise werden die in der nachfolgenden Tabelle aufgeführten Verbindungen synthetisiert. Die meisten sind Öle, die sich wegen ihrer thermischen Instabilität nicht destillieren lassen. Ihre Reinheit wurde durch NMR-Spektrum und Elementaranalyse sichergestellt.

<u>TABELLE</u>

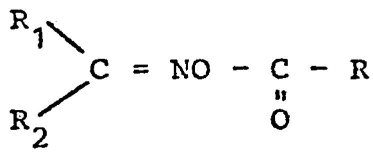

| Beispiel | $R_1$ | $R_2$ | R | $n_D^{25}$ oder Fp. |
|---|---|---|---|---|
| 3 | $-i-C_3H_7$ | $-i-C_3H_7$ | 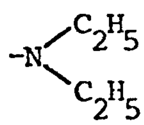 | 1,4588 |
| 4 | " | " | 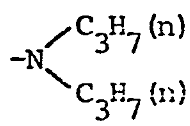 | 1,4600 |
| 5 | " | " | 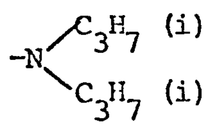 | 1,4614 |
| 6 | " | " | 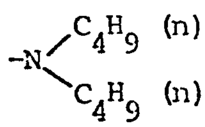 | 1,4588 |
| 7 | " | " | 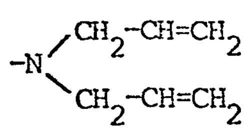 | |
| 8 | " | " | 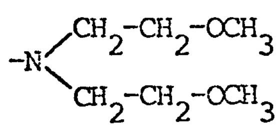 | |
| 9 | " | " | 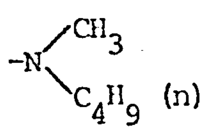 | 1,4605 |
| 10 | " | " | 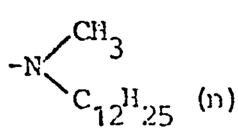 | 1,4615 |

0079537

| Beispiel | $R_1$ | $R_2$ | R | $n_D^{25}$ oder Fp. |
|---|---|---|---|---|
| 11 | $-i-C_3H_7$ | $-i-C_3H_7$ | 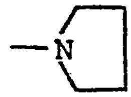 | 1,4790 |
| 12 | " | " | 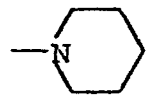 | 1,4819 |
| 13 | " | " | 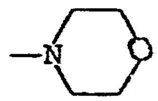 | 1,4808 |
| 14 | " | " | 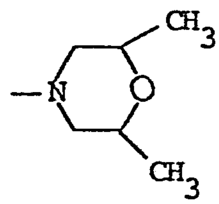 | 1,4705 |
| 15 | " | " | 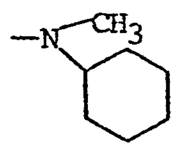 | |
| 16 | " | " | 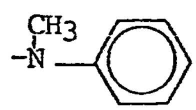 | 1,4808 |
| 17 | " | " | 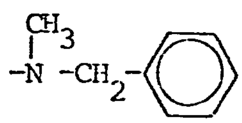 | 1,5128 |
| 18 | " | " | 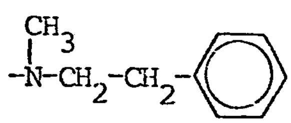 | |
| 19 | $-CH_3$ | " | 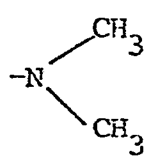 | 1,4583 |
| 20 | " | " | 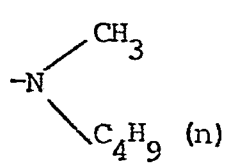 | 1,4595 |
| 21 | " | " | 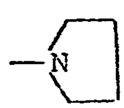 | 1,4827 |

| Beispiel | $R_1$ | $R_2$ | R | $n_D^{25}$ oder Fp. |
|---|---|---|---|---|
| 22 | $-CH_3$ | $-i-C_3H_7$ | 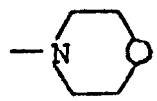 | 1,4875 |
| 23 | $n-C_3H_7$ | " | 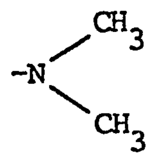 | 1,4608 |
| 24 | " | " | 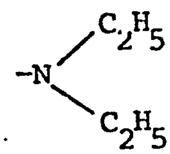 | 1,4600 |
| 25 | $-CH_3$ | $-C(CH_3)_3$ | 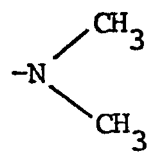 | 1,4588 |
| 26 | " | " | 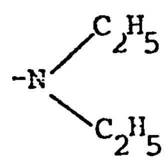 | 1,4581 |
| 27 | " | " | 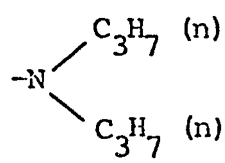 | 1,4605 |
| 28 | " | " | 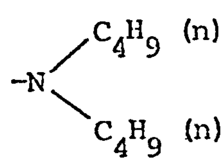 | 1,4594 |
| 29 | " | " | 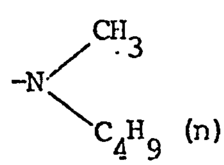 | 1,4600 |
| 30 | " | " | 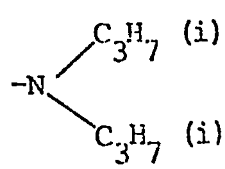 | 1,4625 |

0079537

| Beispiel | $R_1$ | $R_2$ | R | $n_D^{25}$ oder Fp. |
|---|---|---|---|---|
| 31 | $-CH_3$ | $-C(CH_3)_3$ | 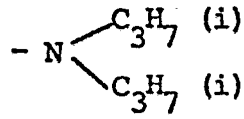 | 1,4610 |
| 32 | " | " | 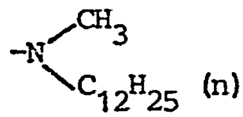 | 1,4610 |
| 33 | " | " | 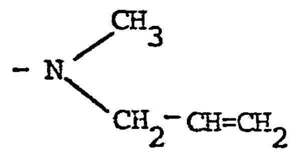 | |
| 34 | " | " | 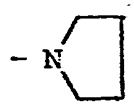 | Fp. 78-80°C |
| 35 | " | " | 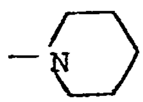 | 1,4800 |
| 36 | " | " | 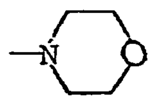 | Fp. 61-63°C |
| 37 | " | " | 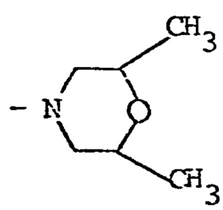 | 1,4796 |
| 38 | " | " | 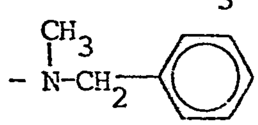 | 1,5156 |
| 39 | " | " | 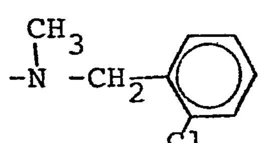 | 1,5285 |
| 40 | " | " | 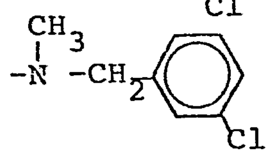 | 1,5262 |
| 41 | " | " | 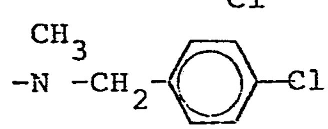 | 1,5262 |

| Beispiel | $R_1$ | $R_2$ | R | $n_D^{25}$ oder Fp. |
|---|---|---|---|---|
| 42 | $CH_3$ | " | 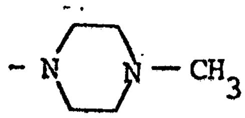 | 1,4870 |
| 43 | 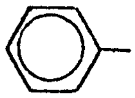 | $i-C_3H_7$ | 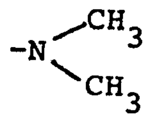 | Fp.88-90°C |
| 44 | " | " | $-N(C_2H_5)_2$ | 1,5171 |
| 45 | " | " | 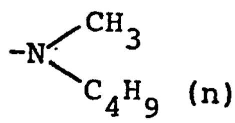 | 1,5145 |
| 46 | " | " | 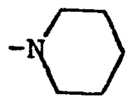 | 1,5445 |
| 47 | " | $-C(CH_3)_3$ | 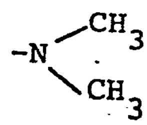 | Fp. 98-99° |
| 48 | " | " | 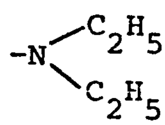 | Fp. 55-57° |
| 49 | 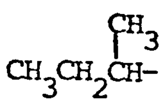 | " | 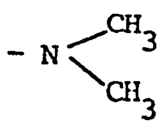 | |
| 50 | " | " | 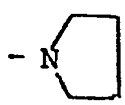 | |
| 51 | 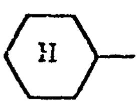 | " | 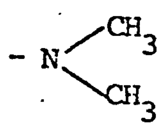 | |
| 52 | 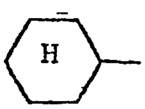 | " | 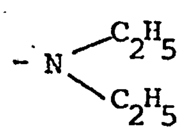 | |
| 53 | $C_2H_5O-CH_2-$ | " | 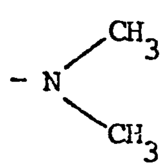 | |

| Beispiel | $R_1$ | $R_2$ | R | $n_D^{25}$ oder Fp. |
|---|---|---|---|---|
| 54 | $C_2H_5OCH_2-$ | " | 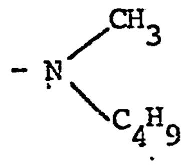 (n) | |
| 55 | $-CH_3$ | 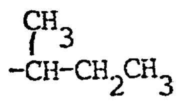 | 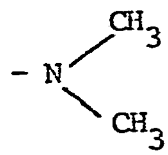 | 1,4630 |
| 56 | " | " | 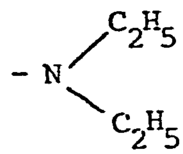 | 1,4615 |
| 57 | " | " | 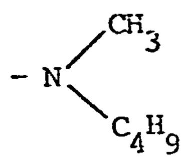 (n) | 1,4616 |

Beispiel I

Samen verschiedener Unkräuter wurden in Plastiktöpfen (Ø 9 cm) in sandigem Lehmboden ausgesät. Die erfindungsgemäßen Verbindungen wurden in Form wässriger Supensionen bzw. Emulsionen mit einer Wasseraufwandmenge von umgerechnet 800 l/ha auf die Oberfläche der Abdeckerde gesprüht. Nach der Behandlung wurden die Töpfe im Gewächshaus unter guten Wachstumsbedingungen (Temperatur 23 - 25°C; Luftfeuchte 60 - 80 % r. F.) aufgestellt. Am Ende einer Standzeit von ca. 3 Wochen erfolgte die optische Bonitur der Pflanzenschutzschädigung im Vergleich zur unbehandelten Kontrolle nach folgendem Schema:

0 = ohne Schädigung
1 = 0 - 20 % Schädigung
2 = 20 - 40 % Schädigung
3 = 40 - 60 % Schädigung
4 = 60 - 80 % Schädigung
5 = 80 - 100 % Schädigung

Tabelle 1

Herbizide Wirkung der erfindungsgemäßen Verbindungen im Vorauflauf

| Verbindung gemäß Herst.-Beispiel | Dosis kg AS / ha | STM | ECG |
|---|---|---|---|
| 1 | 2,5 | 5 | 5 |
| 3 | 2,5 | 5 | 5 |
| 4 | 2,5 | 3 | 4 |
| 5 | 2,5 | 3 | 4 |
| 9 | 2,5 | 4 | 4 |
| 11 | 2,5 | 5 | 2 |
| 14 | 2,5 | 4 | 5 |
| 27 | 2,5 | 4 | 5 |
| 29 | 2,5 | 4 | 5 |
| 30 | 2,5 | 4 | 5 |
| 36 | 2,5 | 4 | 4 |
| 26 | 2,5 | 5 | 5 |
| 25 | 2,5 | 5 | 5 |

Abkürzungen:

STM = Stellaria media
ECG = Echinochloa crus-galli
AS = Aktivsubstanz

Beispiel II

Reis und verschiedenen Reisunkräutern wurden in geschlossenen Plastiktöpfen von 14 cm Durchmesser in Mineralböden ausgesät bzw. ausgepflanzt und als Wasserreis unter warmen Gewächshausbedingungen kultiviert, indem bis 2 - 4 mm über die Bodenoberfläche Wasser in die Töpfe gefüllt wurde. Die Versuchsprodukte wurden in Granulatform in die Töpfe gestreut. Anschließend wurden die Versuchsgefäße 4 Wochen im Gewächshaus gehalten, wobei entsprechend dem Wachstum der Pflanzen der Wasserstand in den Töpfen bei 0,5 - 2 cm Höhe über dem Boden gehalten wurde.

Nach Ablauf von 4 Wochen wurde der Versuch ausgewertet. Die Ergebnisse zeigen die gute Verträglichkeit der neuartigen Herbizide im Reis bei gleichzeitig guter Bekämpfung der verschiedenen Wasserreisunkräuter.

Tabelle 2

Wirkung und Selektivität im Vorauflauf (% Schäden)

| Verbindungen gem. Herst.-Beisp.Nr. | Dosis kg AS/ha | Reis | ECG | CYD | CYS | EOA |
|---|---|---|---|---|---|---|
| 1 | 2 | 0 | 100 | 100 | 100 | 100 |
| | 1 | 0 | 90 | 100 | 95 | 80 |
| 3 | 2 | 0 | 100 | 100 | 100 | 100 |
| | 1 | 0 | 85 | 100 | 100 | 95 |

Abkürzungen:

ECG = Echinochloa crus-galli

CYD = Cyperus difformis

CYS = Cyperus serotinus

EOA = Eleocharis acicularis.

PATENTANSPRÜCHE:

1. Verbindungen der Formel I

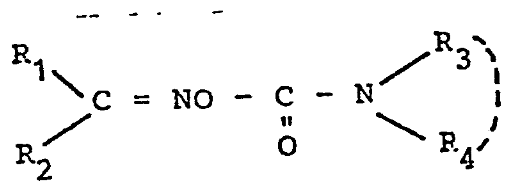

(I)

worin bedeuten:

$R_1$ $(C_1-C_6)$-Alkyl das geradkettig oder verzweigt ist und durch $(C_1-C_4)$-Alkoxy oder $(C_1-C_4)$-Alkoxycarbonyl substituiert sein kann, $(C_3-C_6)$-Cycloalkyl, das durch $(C_1-C_4)$-Alkyl oder $(C_1-C_4)$-Alkoxy substituiert sein kann, oder Phenyl, das durch $(C_1-C_4)$-Alkyl, $(C_1-C_4)$-Alkoxy, Cl oder Br substituiert sein kann,

$R_2$ $(C_3-C_6)$-Alkyl, das geradkettig oder verzweigt ist oder $(C_1-C_4)$-alkoxy-$(C_3-C_6)$-alkyl,

$R_3$ $(C_1-C_6)$-Alkyl, das durch $(C_1-C_4)$-Alkoxy oder $(C_1-C_4)$-Alkoxycarbonyl substituiert sein kann; $(C_1-C_6)$-Alkenyl, $(C_1-C_6)$-Alkinyl, $(C_3-C_6)$-Cycloalkyl, das durch $(C_1-C_4)$-Alkyl oder $(C_1-C_4)$-Alkoxy substituiert sein kann,

$R_4$ $(C_1-C_{12})$-Alkyl, das durch $(C_1-C_4)$-Alkoxy oder $(C_1-C_4)$-Alkoxycarbonyl substituiert sein kann; $(C_1-C_6)$-Alkenyl; Phenyl-, Benzyl- oder Phenethyl, die im aromatischen Kern durch Halogen, $(C_1-C_4)$-Alkyl, $(C_1-C_4)$-Alkoxy, $(C_1-C_4)$-Alkoxycarbonyl, Cyan- oder Trifluormethyl substituiert sein können, oder

$R_3$ und $R_4$ zusammen mit dem benachbarten Stickstoffatom einen 3 - 6-gliedrigen heterocyclischen Ring, der 1 - 2-fach durch $(C_1-C_4)$-Alkyl oder $(C_1-C_4)$-Alkoxy substituiert sein kann und/oder in dem ein oder zwei Ringkohlenstoffatome durch -O-, -S-, $>C=O$, -NH- oder $>N-CH_3$ ersetzt sein können.

2. Verbindungen gemäß Anspruch 1, worin $R_2$ einen verzweigten $(C_3-C_6)$-Alkylrest bedeutet.

3. Verbindungen gemäß Anspruch 1, worin $R_2$ einen Isopropyl-, sek. Butyl- oder tert. Butylrest bedeutet.

4. Verbindungen gemäß einem der Ansprüche 1-3, worin $R_1$ $(C_1-C_4)$-Alkyl oder Phenyl bedeutet.

5. Verbindungen gemäß einem der Ansprüche 1-3, worin $R_1$ Methyl, Isopropyl oder Phenyl bedeutet.

6. Verbindungen gemäß einem der Ansprüche 1-3, worin $R_3$ und $R_4$ unabhängig voneinander $(C_1-C_4)$Alkyl bedeuten oder zusammen eine Tetramethylengruppe darstellen.

7. Verbindungen gemäß Anspruch 1, worin $R_1$ Methyl, Isopropyl oder Phenyl, $R_2$ Isopropyl oder tert. Butyl und $R_3$ und $R_4$ zusammen mit dem Stickstoffatom eine Dimethyl-, Diethyl- oder Methyl-n-butylaminogruppe oder einen Piperidinrest darstellen.

8. Verbindung der Formel

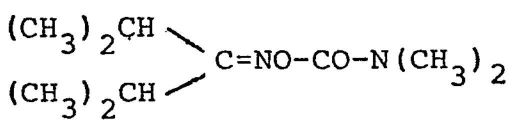

9. Verbindung der Formel

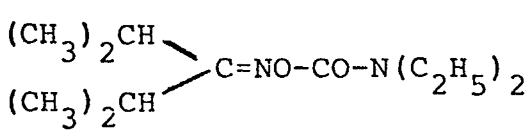

10. Verbindung der Formel

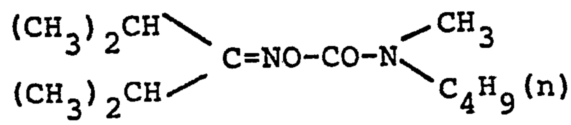

11. Verbindung der Formel

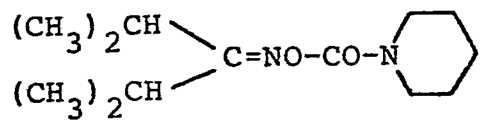

12. Verbindung der Formel

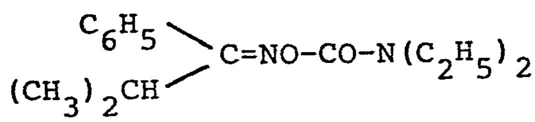

13. Verbindung der Formel

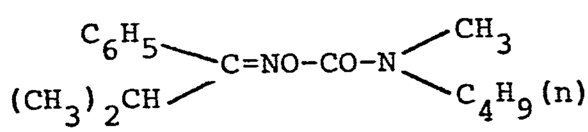

14. Verbindung der Formel

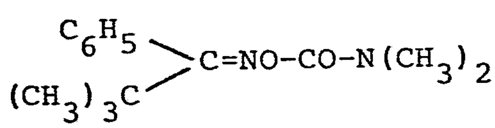

15. Verbindung der Formel

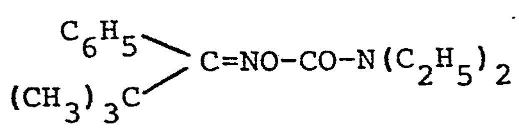

16. Verfahren zur Herstellung von Verbindungen der Formel I, dadurch gekennzeichnet, daß man

a) Verbindungen der Formel

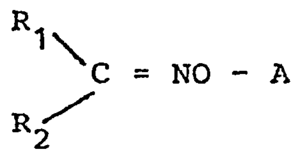

(II)

mit Verbindungen der Formel

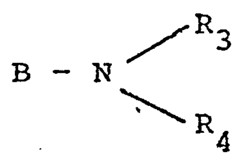

(III),

worin jeweils einer der Reste A und B die Gruppe Hal-CO- und der andere Wasserstoff oder ein Alkali- oder Ammoniumkation darstellt, gegebenenfalls in Anwesenheit von Säureakzeptoren umsetzt, oder

b) Verbindungen der Formel

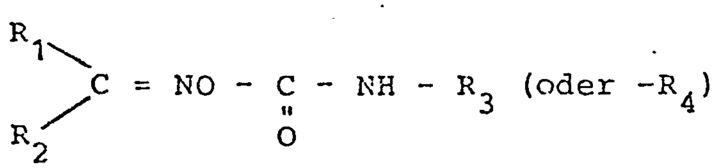

(IV)

alkyliert.

17. Herbizide Mittel, gekennzeichnet durch einen Gehalt an einer Verbindung der Formel I.

18. Herbizide Mittel, gekennzeichnet durch einen Gehalt an einer Verbindung gemäß einem der Ansprüche 2-7.

19. Herbizide Mittel, gekennzeichnet, durch einen Gehalt an einer Verbindung gemäß einem der Ansprüche 8-15.

20. Verwendung von Verbindungen der Formel I zur Bekämpfung von unerwünschtem Pflanzenwuchs.

21. Verfahren zur Bekämpfung unerwünschten Pflanzenwuchses, dadurch gekennzeichnet, daß man eine wirksame Menge einer Verbindung der Formel I auf die Pflanzen bzw. die von ihnen befallene Fläche aufbringt.

22. Verfahren zur Bekämpfung unerwünschten Pflanzenwuchses, dadurch gekennzeichnet, daß man eine wirksame Menge einer Verbindung gemäß einem der Ansprüche 2-7 auf die unerwünschten Pflanzen bzw. die von ihnen befallene Fläche aufbringt.

23. Verfahren zur Bekämpfung unerwünschten Pflanzenwuchses, dadurch gekennzeichnet, daß man eine wirksame Menge einer Verbindunge gemäß einem der Ansprüche 8-15 auf die unerwünschten Pflanzen bzw. die von ihnen befallene Fläche aufbringt.

Europäisches Patentamt

# EUROPÄISCHER RECHERCHENBERICHT

0079537
Nummer der Anmeldung

EP 82 11 0188

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-2 113 752 (AGRIPAT S.A.)<br>* Seite 28 : Verbindung 10 * | 1,16 | C 07 C 131/00<br>C 07 D 295/20<br>C 07 D 265/30<br>A 01 N 37/18 |
| A | DE-A-1 618 361 (FARBENFABRIKEN BAYER)<br>* Beispiele 8, 10 * | 1,11, 16 | |
| A | US-A-3 376 342 (SMITH KLINE & FRENCH LABORATORIES)<br>* Beispiel 7 * | 1,2,5, 16 | |
| A | GB-A-1 207 788 (SHELL INTERNATIONALE RESEARCH MATTSCHAPPIJ)<br>* Beispiel 1 * | 1,5,16 ,17-23 | |
| | ----- | | RECHERCHIERTE SACHGEBIETE (Int. Cl. 3) |
| | | | C 07 C 131/00<br>C 07 C 135/00 |

Der vorliegende Recherchenbericht wurde für alle Patentansprüche erstellt.

| Recherchenort | Abschlußdatum der Recherche | Prüfer |
|---|---|---|
| BERLIN | 28-01-1983 | BREW C.H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82